(19) Europäisches Patentamt / European Patent Office / Office européen des brevets

(11) **EP 3 747 368 B1**

(12) **EUROPEAN PATENT SPECIFICATION**

(45) Date of publication and mention
of the grant of the patent:
**07.01.2026  Bulletin 2026/02**

(51) International Patent Classification (IPC):
**A61B 8/12** *(2006.01)*      **C08L 101/00** *(2006.01)*
**A61B 8/00** *(2006.01)*

(21) Application number: **19748003.1**

(22) Date of filing: **25.01.2019**

(52) Cooperative Patent Classification (CPC):
**A61B 8/4444; A61B 8/12; C08L 101/00**

(86) International application number:
**PCT/JP2019/002471**

(87) International publication number:
**WO 2019/151135 (08.08.2019 Gazette 2019/32)**

(54) **ULTRASONIC PROBE AND RESIN COMPOSITION FOR ULTRASONIC PROBE**

ULTRASCHALLSONDE UND HARZZUSAMMENSETZUNG FÜR ULTRASCHALLSONDE

SONDE ULTRASONORE ET COMPOSITION DE RÉSINE POUR SONDE ULTRASONORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **30.01.2018   JP 2018013748**

(43) Date of publication of application:
**09.12.2020   Bulletin 2020/50**

(73) Proprietor: **FUJIFILM Corporation
Tokyo 106-8620 (JP)**

(72) Inventor: **NAKAI, Yoshihiro
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(56) References cited:
EP-A2- 1 862 227        JP-A- 2001 340 342
JP-A- 2017 056 142      JP-A- H0 987 531
US-A1- 2014 249 419     US-A1- 2016 338 666

## Description

### BACKGROUND OF THE INVENTION

1. Field of the Invention

The present invention relates to an ultrasound probe and use of a resin composition for producing a sheath material for an ultrasound probe.

2. Description of the Related Art

An ultrasound probe is generally a medical apparatus with which an ultrasound probe head is brought into contact with the surface of a subject to irradiate the subject with ultrasonic waves, receive reflected waves (echoes) from the interior of the subject, and enable observation of the interior of the subject. The received reflected waves are converted to electrical signals, which help to visualize the interior of the subject for observation.

Recently, it has become practice to insert a downsized ultrasound probe into an endoscope forceps opening and then into body cavities such as the rectum and the bronchi to transmit and receive ultrasonic waves within the body cavities to thereby examine the interior of a body in more detail.

For a long time, resin materials, such as polyethylene and polytetrafluoroethylene, have been used as sheath materials forming covers of such downsized ultrasound probes. However, with these resin materials, the amount of reflected ultrasonic waves that are supposed to be transmitted is large. As a result, the provision of sufficient sensitivity has been limited.

Various materials serving as sheath materials that suppress the reflection of ultrasonic waves have been studied. For example, JP2001-340342A discloses the use of a blended resin as a sheath material at least for an ultrasonic transmission window, the blended resin containing a mixture of polyurethane and a copolymer resin that contains a polyamide block and a polyether ester block. JP2001-340342A also discloses that with the disclosed technique, an ultrasound probe becomes less prone to buckling even when inserted into an endoscope channel (forceps opening) or the like, and multiple reflections or the like in the vicinity of the ultrasonic transmission window can be suppressed. US 2014/249419 A1 relates to a method for manufacturing an ultrasound probe which includes a piezoelectric element and an acoustic matching layer provided by lamination of two or more matching materials on a front surface of the piezoelectric element. US 2016/0338666 A1 is related to an acoustic lens for an ultrasound probe comprising a vulcanized molded rubber composition containing silicone rubber and metal oxide particles dispersed in the silicone rubber.

### SUMMARY OF THE INVENTION

The present invention is defined by the ultrasound probe according to claim 1 and the use of a resin composition according to claim 8.

In a downsized ultrasound probe, a sheath material serves as a contact surface when the ultrasound probe is moved in an endoscope forceps opening or in body cavities. Thus, the sheath material desirably exhibits properties of enabling the ultrasound probe to be moved smoothly while in contact with the inner wall of an endoscope forceps opening, the inner walls of body cavities, or the like.

In every use, an ultrasound probe is subjected to a disinfection treatment or a sterilization treatment using a chemical solution or the like and is reused. When a sheath comes in contact with moisture through such a chemical solution treatment and undergoes hygroscopic elongation, the fixed state of a shaft, an ultrasonic oscillator, or the like is adversely affected, and a space is formed, for example, between an acoustic medium and the sheath, which may potentially impair the propagation of ultrasonic waves. Thus, the sheath material desirably exhibits properties of being resistant to hygroscopic elongation.

In view of ultrasonic properties, the sheath material desirably exhibits properties of enabling ultrasonic waves transmitted from the probe and returned from an affected area to be transmitted therethrough with as little attenuation as possible.

Thus, an object of the present invention is to provide an ultrasound probe that is capable of being moved in an endoscope forceps opening, body cavities, or the like smoothly with low friction (i.e., that has excellent abrasion resistance), that is less prone to buckling while, for example, being bent sharply (i.e., that has excellent bending rigidity), that is sufficiently resistant to hygroscopic elongation, and that has excellent ultrasonic properties. Another object of the present invention is to provide a resin composition suitable as a sheath material for the ultrasound probe.

As a result of intensive research in light of the objects, the inventors have found that the objects can be achieved by using a thermoplastic resin as a material forming a sheath of an ultrasound probe, the thermoplastic resin including resin particles that have a particle diameter within a specific range dispersed therein, thereby completing the present invention.

The ultrasound probe according to the present invention is capable of being moved in an endoscope forceps opening, body cavities, or the like smoothly with low friction (i.e., has excellent abrasion resistance), is less prone to buckling while, for example, being bent sharply (i.e., has excellent bending rigidity), is sufficiently resistant to hygroscopic elongation, and has excellent ultrasonic properties. The resin composition used in the present invention is suitable as a sheath material for the ultrasound probe according to the present invention.

**BRIEF DESCRIPTION OF THE DRAWINGS**

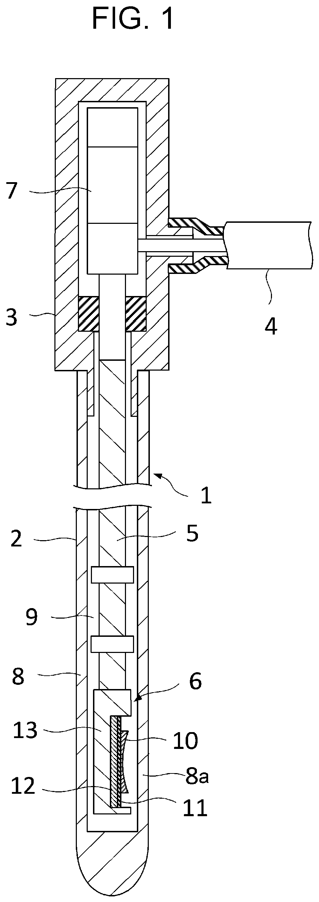

Fig. 1 is a schematic sectional view of an example of an ultrasound probe.

**DESCRIPTION OF THE PREFERRED EMBODIMENTS**

An ultrasound probe according to the present invention is not particularly limited as long as a sheath includes a thermoplastic resin (hereafter also referred to as a "resin particle-containing thermoplastic resin") formed by dispersing resin particles having a particle diameter of 0.1 to 100 $\mu$m, and a common structure of an ultrasound probe can be adopted except that the sheath includes a resin particle-containing thermoplastic resin. That is, the ultrasound probe according to the present invention includes a structure that has a shaft having an ultrasonic oscillator on a front end thereof and a sheath (cover) covering the shaft.

An example of the structure of an ultrasound probe 1 according to the present invention will be described in detail with reference to Fig. 1.

As illustrated in Fig. 1, the ultrasound probe 1 has a flexible insert section 2 to be inserted into body cavities or into an endoscope forceps opening, a grip section 3 disposed on the rear end of the insert section 2, and a cable section 4 extending from this grip section 3. The cable section 4 is linked to an ultrasonic monitoring apparatus (not illustrated) that transmits a drive signal to an ultrasonic oscillator 6 and that amplifies a reflected wave (an electrical signal) from a subject which has been received by the ultrasonic oscillator 6 to display an ultrasonic tomogram.

A shaft 5 is inserted into the insert section 2 of the ultrasound probe 1, and the ultrasonic oscillator 6 is disposed on the front end of the shaft 5. The rear end of the shaft 5 is connected to a motor 7 disposed within the grip section 3, and when the motor 7 is rotated, the ultrasonic oscillator 6 can be rotationally driven together with the shaft 5.

An acoustic medium 9, such as water or fluid paraffin that transmits ultrasonic waves, is filled in between a sheath 8, which is a cover of the insert section 2, and the ultrasonic oscillator 6. A portion of the sheath 8 that faces the ultrasonic oscillator 6 forms an ultrasonic transmission window 8a.

The ultrasonic oscillator 6 has a plate-like piezoelectric oscillator 11 having piezoelectric characteristics that enable electroacoustic conversion, an acoustic lens 10 disposed on the front surface of the piezoelectric oscillator 11, and a backing layer 12 disposed on the rear surface of the piezoelectric oscillator 11. This backing layer 12 portion is adhesively fixed to a housing 13, and this housing 13 is mounted to the front end of the shaft 5.

In the ultrasound probe according to the present invention, a material forming the sheath includes a resin particle-containing thermoplastic resin. Thus, the provision of an ultrasound probe that is capable of being moved in an endoscope forceps opening, body cavities, or the like smoothly with low friction, that is less prone to buckling, that is sufficiently resistant to hygroscopic elongation, and that has excellent ultrasonic properties is realized. Hereafter, the sheath which is a structure characteristic of the present invention will be described.

Sheath

The sheath used in the present invention contains a thermoplastic resin serving as a base resin and resin particles having a particle diameter of 0.1 to 100 $\mu$m.

Thermoplastic Resin

The thermoplastic resin used as a material forming the sheath is not particularly limited. Examples of the thermoplastic resin include polyamide resin, polyester resin, polyurethane resin, polyolefin resin, polystyrene resin, and acrylic resin, and two or more of the foregoing may be used in combination.

Polyamide Resin

A wide variety of common polyamide resins usable as sheath materials for ultrasound probes can be used as the polyamide resin. Examples thereof include crystalline polyamides, amorphous polyamides, and polyamide elastomers.

The crystalline polyamides are not particularly limited, and examples thereof include aliphatic polyamides and aromatic polyamides.

Examples of the aliphatic polyamides include poly(ε-caproamide) (polyamide 6), polytetramethylene adipamide (polyamide 46), polyhexamethylene adipamide (polyamide 66), polycaproamide/polyhexamethylene adipamide copolymers (polyamide 6/66), polyundecamide (polyamide 11), polycaproamide/polyundecamide copolymers (polyamide 6/11), polydodecamide (polyamide 12), polycaproamide/polydodecamide copolymers (polyamide 6/12), polyhexamethylene sebacamide (polyamide 610), polyhexamethylene dodecamide (polyamide 612), polyundecamethylene adipamide (polyamide 116), and mixtures or copolymers of the foregoing.

Examples of the aromatic polyamides include polyhexamethylene isophthalamide (polyamide 6I), polyhexamethylene terephthalamide (polyamide 6T), polyhexamethylene terephthalamide/polyhexamethylene isophthalamide copolymers (polyamide 6T/6I), polycaproamide/polyhexamethylene terephthalamide copolymers (polyamide 6/6T), polycaproamide/polyhexamethylene isophthalamide copolymers (polyamide 6/6I), polyhexamethylene adipamide/polyhexamethylene terephthalamide copolymers (polyamide 66/6T), polyhexamethylene adipamide/polyhexamethylene isophthalamide copolymers (polyamide 66/6I), polytrimethylhexamethylene terephthalamide (polyamide TMDT), poly-bis(4-aminocyclohexyl)methanedodecamide (polyamide PACM12), polybis(3-methyl-4-aminocyclohexyl)methanedodecamide (nylon dimethyl PACM12), poly(meta-xylylene adipamide) (polyamide MXD6), polydecamethylene terephthalamide (polyamide 10T), polyundecamethylene terephthalamide (polyamide **11T),** and mixtures or copolymers of the foregoing.

Examples of the amorphous polyamides include polycondensates of isophthalic acid/terephthalic acid/1,6-hexanediamine/bis(3-methyl-4-aminocyclohexyl)methane, polycondensates of terephthalic acid/2,2,4-trimethyl-1,6-hexanediamine/2,4,4-trimethyl-1,6-hexanediamine, polycondensates of isophthalic acid/bis(3-methyl-4-aminocyclohexyl)methane/ω-laurolactam, polycondensates of isophthalic acid/terephthalic acid/1,6-hexanediamine, polycondensates of isophthalic acid/2,2,4-trimethyl-1,6-hexanediamine/2,4,4-trimethyl-1,6-hexanediamine, polycondensates of isophthalic acid/terephthalic acid/2,2,4-trimethyl-1,6-hexanediamine/2,4,4-trimethyl-1,6-hexanediamine, polycondensates of isophthalic acid/bis(3-methyl-4-aminocyclohexyl)methane/ω-laurolactam, and polycondensates of isophthalic acid, terephthalic acid, and diamine components other than mentioned above.

The polyamide elastomers are, for example, multiblock copolymers of polyamide serving as a hard segment and polyether or polyester serving as a soft segment. Examples of the hard segment include polyamides 6, 66, 610, 11, and 12. Examples of the polyether serving as a soft segment include polyethylene glycol, diolpoly(oxytetramethylene) glycol, poly(oxypropylene) glycol, and examples of the polyester serving as a soft segment include poly(ethylene adipate) glycol and poly(butylene-1,4-adipate) glycol.

Polyester Resin

Examples of the polyester resin include polyester resins formed of a dicarboxylic acid component and a diol component and polyester resins formed of a hydroxycarboxylic acid component.

Examples of the dicarboxylic acid component include terephthalic acid, isophthalic acid, phthalic acid, 2,6-naphthalenedicarboxylic acid, 5-sodiumsulfoisophthalic acid, oxalic acid, succinic acid, adipic acid, sebacic acid, azelaic acid, dodecanedioic acid, dimer acid, maleic anhydride, maleic acid, fumaric acid, itaconic acid, citraconic acid, mesaconic acid, and cyclohexanedicarboxylic acid.

Examples of the diol component include ethylene glycol, diethylene glycol, 1,3-propanediol, 1,4-butanediol, neopentyl glycol, 1,6-hexanediol, cyclohexanedimethanol, triethylene glycol, polyethylene glycol, polypropylene glycol, polytetramethylene glycol, ethylene oxide adducts of bisphenol A, and ethylene oxide adducts of bisphenol S.

Examples of the hydroxycarboxylic acid component include ε-caprolactone, lactic acid, and 4-hydroxybenzoic acid.

The polyester resin may be a homopolymer or a copolymer of the foregoing components, and may further contain a small amount of a trifunctional compound component such as trimellitic acid, trimesic acid, pyromellitic acid, trimethylol propane, glycerol, or pentaerythritol. As the polyester resin, two or more homopolymers or copolymers of the foregoing components may be used in combination.

The polyester resin is also preferably a polyester elastomer. The polyester elastomer is not particularly limited, and a wide variety of polyester elastomers applicable to ultrasound probes can be used. For example, as disclosed in JP1999-92636A (JP-H11-92636A) and the like, a block copolymer of a high-melting-point polyester segment (hard segment) and a low-melting-point polymer segment (soft segment) having a molecular weight of 400 to 6,000 can be used.

Polyurethane Resin

The polyurethane resin is not particularly limited, and a wide variety of polyurethane resins applicable to ultrasound probes can be used. For example, polyurethane resins such as carbonate-based polyurethane resins, ether-based polyurethane resins, and ester-based polyurethane resins can be used. The polyurethane resin is also preferably a polyurethane elastomer. The polyurethane elastomer can be appropriately selected according to the purpose.

An example thereof is an elastomer including a structural unit that is made of a hard segment formed of a low-molecular glycol and a diisocyanate and a soft segment formed of a high-molecular (long-chain) diol and a diisocyanate.

Examples of the high-molecular (long-chain) diol include polypropylene glycol, polytetramethyleneoxide, poly(1,4-butylene adipate), poly(ethylene-1,4-butylene adipate), polycaprolactone, poly(1,6-hexylene carbonate), and poly(1,6-hexylene-neopentylene adipate). The number average molecular weight of the high-molecular (long-chain) diol is preferably 500 to 10,000.

As the low-molecular glycol, a short-chain diol such as ethylene glycol, propylene glycol, 1,4-butanediol, or bisphenol A can be used. The number average molecular weight of the short-chain diol is preferably 48 to 500.

Examples of commercially available polyurethane resins include PANDEX T-2185 or T-2983N (both manufactured by DIC Corporation), Miractran (manufactured by Nippon Miractran Co., Ltd.), Elastollan (manufactured by BASF Japan Ltd.), Resamine (manufactured by Dainichiseika Color and Chemicals Mfg. Co., Ltd.), Pellethane (manufactured by Dow Chemical Japan Ltd.), Iron Rubber (manufactured by NOK Corporation), and Mobilon (manufactured by Nisshinbo Chemical, Inc.). The examples further include Isoplast (manufactured by The Lubrizol Corporation), Tecoflex (manufactured by The Lubrizol Corporation), SUPERFLEX 830, 460, 870, 420, or 420NS (polyurethane manufactured by Daiichi Kogyo Seiyaku Co., Ltd.), Hydran AP-40F, WLS-202, or HW-140SF (polyurethane manufactured by Dainippon Ink and Chemicals, Inc.), OLESTER UD500 or UD350 (polyurethane manufactured by Mitsui Chemicals, Inc.), and TAKELAC W-615, W-6010, W-6020, W-6061, W-405, W-5030, W-5661, W-512A-6, W-635, or WPB-6601 (manufactured by Mitsui Chemicals, Inc.).

Polyolefin Resin

The polyolefin resin is not particularly limited, and a wide variety of polyolefin resins applicable to ultrasound probes can be used. Examples thereof include homopolymers or copolymers of an $\alpha$-olefin having 2 to 20 carbon atoms such as ethylene, propylene, 1-butene, 1-hexene, or 4-methylpentane. The examples also include copolymers of an $\alpha$-olefin and a non-conjugated diene having 2 to 20 carbon atoms such as dicyclopentadiene, 1,4-hexadiene, cyclooctadiene, methylene norbornene, ethylidene norbornene, butadiene, or isoprene. The examples also include ethylene-$\alpha$-olefin copolymer rubber, ethylene-$\alpha$-olefin-non-conjugated diene copolymer rubber, propylene-$\alpha$-olefin copolymer rubber, and butene-$\alpha$-olefin copolymer rubber. As the polyolefin resin, an ethylene-(meth)acrylic acid copolymer, an ethylene-(meth)acrylic acid ester-(meth)acrylic acid copolymer, an ethylene-vinyl acetate copolymer, an ethylene-vinyl acetate-(meth)acrylic acid copolymer, an ethylene-propylene-(meth)acrylic acid copolymer, an ethylene-propylene-(meth)acrylic acid ester-(meth)acrylic acid copolymer, an ethylene-maleic anhydride copolymer, an ethylene-(meth)acrylic acid ester-maleic anhydride copolymer, a copolymer of ethylene, butene, maleic anhydride, and/or (meth)acrylic acid, a copolymer of propylene, butene, maleic anhydride, and/or (meth)acrylic acid, an ethylene-vinyl chloride copolymer, or the like can also be used.

Polystyrene Resin

The polystyrene resin is not particularly limited, and a wide variety of polystyrene resins applicable to ultrasound probes can be used. The term "polystyrene resin" refers to a resin including 50% by mass or more of a styrene component. In the present invention, the polystyrene resin may be used alone or in a combination of two or more. As used herein, the term "styrene component" refers to a structural unit derived from a monomer having a styrene skeleton in the structure thereof.

Examples of the polystyrene resin include homopolymers of a styrene compound and copolymers of two or more styrene compounds. As used herein, the term "styrene compounds" refers to compounds having a styrene skeleton in the structure thereof, including styrene, and in addition, compounds having a substituent introduced into a moiety of styrene other than an ethylenically unsaturated bond moiety. Examples of the styrene compounds include styrene; alkylstyrenes such as $\alpha$-methylstyrene, o-methylstyrene, m-methylstyrene, p-methylstyrene, 1,3-dimethylstyrene, 2,4-dimethylstyrene, o-ethylstyrene, p-ethylstyrene, and tert-butylstyrene; and substituted styrenes having a hydroxyl group, an alkoxy group, a carboxyl group, a halogen, or the like introduced into the benzene nucleus of styrene, such as hydroxystyrene, tert-butoxystyrene, vinylbenzoic acid, o-chlorostyrene, and p-chlorostyrene.

Components other than the styrene component that can be included in the polystyrene are not particularly limited. That is, the polystyrene resin may be a styrene-diene copolymer or a styrene-polymerizable unsaturated carboxylic acid ester copolymer. A mixture of polystyrene and synthetic rubber (e.g., polybutadiene and polyisoprene) can also be used. A so-called styrene elastomer can also be suitably used.

The polystyrene resin may be hydrogenated (may be a hydrogenated polystyrene resin). The hydrogenated polystyrene is not particularly limited, but a hydrogenated styrene-diene copolymer such as a hydrogenated styrene-butadiene-styrene block copolymer (SEBS), which is a resin formed by hydrogenating a styrene-butadiene-styrene block copolymer (SBS), a hydrogenated styrene-isoprene-styrene block copolymer (SEPS), which is a resin formed by hydrogenating a styrene-isoprene-styrene block copolymer (SIS), or the like is preferable. The hydrogenated polystyrene resin may be used alone or in a combination of two or more.

Acrylic Resin

The acrylic resin is not particularly limited, and a wide variety of acrylic resins applicable to ultrasound probes can be used. An example of the acrylic resin is a polymer formed by polymerizing a raw material monomer having a (meth) acrylic acid ester as a main component. As used herein, the term "(meth)acrylic acid ester" is meant to include both acrylic acid ester and methacrylic acid ester. That is, the term "(meth)acrylic acid ester" refers to at least one of an acrylic acid ester or a methacrylic acid ester. Examples of the (meth)acrylic acid ester include methyl methacrylate, methyl acrylate, ethyl methacrylate, ethyl acrylate, butyl acrylate, methoxyethyl acrylate, and ethoxyethyl acrylate. Glycidyl methacrylate, allyl glycidyl ether, or the like may be used as a raw material for a crosslinking monomer. As needed, the foregoing (meth)acrylic acid esters may be copolymerized with acrylic acid, methacrylic acid, or the like.

The (meth)acrylic acid ester may be copolymerized with acrylonitrile or the like. Specifically, an acrylonitrile-butyl acrylate copolymer, an acrylonitrile-butyl acrylate-ethyl acrylate copolymer, an acrylonitrile-butyl acrylate-glycidyl methacrylate copolymer, or the like can be exemplified.

The thermoplastic resin preferably includes at least one of a polyamide resin, a polystyrene resin, a polyolefin resin, or an acrylic resin in view of further suppressing ultrasonic attenuation. The thermoplastic resin preferably includes a polyamide resin also in view of further improving bending rigidity. Bending rigidity is a physical property needed for imparting to an ultrasound probe a physical property of being less prone to buckling while being bent sharply when, for example, the ultrasound probe is inserted into an endoscope forceps opening.

Among the above-described polyamide resins, at least one of polyamide **11,** polyamide 12, an amorphous polyamide, or a polyamide elastomer is preferably used in view of realizing the suppression of hygroscopic elongation and higher ultrasonic properties.

The percentage of the polyamide resin contained in the thermoplastic resin that forms the sheath is preferably 50% by mass or more, more preferably 70% by mass or more, even more preferably 80% by mass or more, and particularly preferably 90% by mass or more. Resin Particles

The resin particles serving as a material forming the sheath have a particle diameter in the range of 0.1 to 100 $\mu$m. In the present invention, the term "particle diameter" refers to a volume average particle diameter. The volume average particle diameter is determined as follows.

The resin particles are added to methanol so as to be contained in an amount of 0.5% by mass, and the mixture is sonicated for 10 minutes to disperse the resin particles. The particle size distribution of the particles thus treated is measured with a laser diffraction and scattering particle size distribution analyzer (product name: LA950V2, manufactured by Horiba, Ltd.), and the measured volumetric median diameter is determined to be the particle diameter. The median diameter corresponds to the particle diameter at 50% in the cumulative distribution when the particle size distribution is represented in cumulative form. The resin particles more preferably have a distribution in which 70% by weight or more of the particles falls in the range of 5 $\mu$m around the above-described average particle diameter.

The ultrasound probe according to the present invention is capable of being moved in an endoscope forceps opening, body cavities, or the like smoothly with low friction because the sheath contains the resin particles having a specific particle diameter in addition to the thermoplastic resin. Probably, one factor here is that the moderate unevenness of a sheath surface resulting from the resin particles enables a smaller contact area with the interior of an endoscope forceps opening, the interior of body cavities, or the like. Furthermore, because the particles are resin particles, sufficient compatibility with the thermoplastic resin serving as a base resin of the sheath can be achieved. That is, because the sheath contains the thermoplastic resin and the resin particles, low friction can be achieved, detachment of the resin particles can be effectively suppressed, and abrasion resistance is improved.

Additionally, the resin particles in the sheath do not adversely affect the bending rigidity of the base resin, and thus enable sufficient bending rigidity of the sheath.

The particle diameter of the resin particles is preferably 1 to 50 $\mu$m, more preferably 2 to 30 $\mu$m, even more preferably 3 to 20 $\mu$m in view of further improving abrasion resistance. The shape of the resin particles is preferably a substantially spherical shape in view of abrasion resistance and ultrasonic properties and is more preferably as close as possible to a fully spherical shape. The term "spherical shape" also refers to an oval sphere.

The sheath is preferably formed with the resin particles homogenously dispersed in the thermoplastic resin. To cause such a state, usually, the resin particles and the thermoplastic resin are kneaded and molded at or above the melting point of the thermoplastic resin. Thus, when the resin particles melt during melt-kneading, the particle shape thereof cannot be maintained. Accordingly, the resin particles need to be able to maintain the particle shape thereof even when melt kneading is performed. Thus, the material forming the resin particles is preferably a resin having a high melting point. The high-melting-point resin is not particularly limited as long as it has a desired high melting point. For example, polytetrafluoroethylene, poly(vinylidene fluoride), poly(phenylene sulfide), polyethersulfone, polyamide-imide, or the like can be used. These resins may have a (meth)acrylic acid ester component, a styrene component, or the like in a polymer chain thereof and preferably include at least one of a (meth)acrylic acid ester component or a styrene component.

The term "(meth)acrylic acid ester component" is meant to include to both acrylic acid ester and methacrylic acid ester components. That is, the term "(meth)acrylic acid ester component" refers to at least one of an acrylic acid ester component or a methacrylic acid ester component.

By introducing a crosslinked structure into the resin particles so as to make the resin particles crosslinked resin particles (i.e., by making the resin (polymer) forming the resin particles have a crosslinked structure), the resin particles can be kept from melting in the heat.

The crosslinked resin particles usable in the present invention are typically obtainable by polymerizing a non-crosslinking monomer (monomer having one ethylenically unsaturated bond) and a crosslinking monomer (monomer having two or more ethylenically unsaturated bonds). Other copolymerizable monomers than the foregoing monomers can also be used.

Examples of the non-crosslinking monomer include non-crosslinking vinyl monomers such as acrylic monomers, styrene monomers, and acrylonitrile monomers, and olefin monomers.

Examples of the acrylic monomers include methyl acrylate, ethyl acrylate, propyl acrylate, butyl acrylate, 2-ethylhexyl acrylate, methyl methacrylate, ethyl methacrylate, propyl methacrylate, butyl methacrylate, 2-ethylhexyl methacrylate, and phenyl methacrylate, and these can be used alone or in a combination of two or more. Among these, methyl methacrylate is preferably used as a non-crosslinking monomer.

As the styrene monomers, styrene, alkylstyrenes such as $\alpha$-methylstyrene, methylstyrene (vinyltoluene), and ethylstyrene, and halogenated styrenes such as brominated styrenes can be used. Among these, styrene is preferable.

As the acrylonitrile monomers, acrylonitrile and methacrylonitrile can be used.

As the olefin monomers, ethylene, various kinds of norbornene compounds, and the like can be used.

Examples of the above-described "other copolymerizable monomers" include glycidyl methacrylate, N-methylmaleimide, and maleic anhydride.

Examples of the above-described crosslinking monomers include divinylbenzene, allyl methacrylate, triallyl cyanurate, triallyl isocyanate, ethylene glycol di(meth)acrylate, diethylene glycol di(meth)acrylate, propylene glycol di(meth)acrylate, 1,6-hexanediol di(meth)acrylate, trimethylolpropane tri(meth)acrylate, pentaerythritol tetra(meth)acrylate, bisphenol A di(meth)acrylate, dicyclopentanyl di(meth)acrylate, and dicyclopentenyl di(meth)acrylate.

The resin of the crosslinked resin particles preferably includes at least one of a (meth)acrylic acid ester component or a styrene component as a component of the resin.

The content of the resin particles in the sheath is preferably 0.2% to 20% by mass, more preferably 0.5% to 15% by mass, even more preferably 1% to 12% by mass, still even more preferably 1% to 10% by mass, particularly preferably 1.5% to 8% by mass, and especially preferably 2% to 6 % by mass. When the content of the resin particles in the sheath is 0.2% by mass or more, higher abrasion resistance is obtainable. When the content of the resin particles in the sheath is 20% by mass or less, hygroscopic elongation can be further suppressed and higher ultrasonic properties can be achieved.

In the sheath of the ultrasound probe according to the present invention, as long as a combination of the above-described thermoplastic resin and the above-described resin particles is contained, components other than the above-described thermoplastic resin and other than the above-described resin particles ("other components") may also be contained to the extent that does not impair the advantageous effects of the present invention. Examples of such "other components" include colorants such as pigments and dyes, heat stabilizers, antioxidants, plasticizers, lubricants, mold release agents, and antistatic agents.

The total content of the above-described thermoplastic resin and the above-described resin particles in the sheath of the ultrasound probe according to the present invention is preferably 70% by mass or more, more preferably 80% by mass or more, and even more preferably 90% by mass or more.

Production of Sheath

A common method can be used to produce a sheath used for the ultrasound probe according to the present invention except that the resin particles are incorporated into the sheath. For example, a desired amount of the thermoplastic resin and the resin particles are mixed, and the mixture is kneaded at a temperature which is equal to or above the melting point of the thermoplastic resin and at which the resin particles do not melt, to obtain a resin composition. The method for kneading is not particularly limited as long as it enables homogenous mixing of all components. For example, kneading can be performed with a mixer or a kneading machine (e.g., a kneader, a pressure kneader, a Banbury mixer (continuous kneader), or a double-roll kneading apparatus).

By subjecting this resin composition to injection molding, extrusion molding, pressure molding, or the like, a sheath having a desired shape is obtainable.

In the ultrasound probe according to the present invention, structures or members commonly used for ultrasound probes can be used without particular limitation as structures or members other than the above-described sheath.

Hereafter, the present invention will be described in further detail on the basis of Examples, but these Examples are not intended to limit the present invention.

**EXAMPLES**

Materials

Thermoplastic Resin (A)

(A-1)
Polyamide 6 ("Novamid ST-220", manufactured by DSM Engineering Plastics, Inc.)
(A-2)
Polyamide 66 ("Novamid 3010SR", manufactured by DSM Engineering Plastics, Inc.)
(A-3)
Polyamide 11 ("Rilsan BMN O", manufactured by Arkema S.A.)
(A-4)
Polyamide 12 ("Vestamid L1940", manufactured by Daicel-Evonik Ltd.)
(A-5)
Polyamide 9T ("Genestar N1000A", manufactured by Kuraray Co., Ltd.)
(A-6)
Polyamide 46 ("Stanyl TW341", manufactured by DSM Engineering Plastics, Inc.)
(A-7)
Polyamide 610 ("Vestamid HS16", manufactured by Daicel-Evonik Ltd.)
(A-8)
Polyamide 1010 ("Vestamid DS16", manufactured by Daicel-Evonik Ltd.)
(A-9)
Amorphous polyamide ("Trogamid CX7323", manufactured by Daicel-Evonik Ltd.)
(A-10)
Polyamide elastomer ("Pebax 7233", manufactured by Arkema S.A.)
(A-11)
Polyamide elastomer ("Pebax Rnew72R53", manufactured by Arkema S.A.)
(A-12)
Polyester elastomer ("Hytrel 7247", manufactured by Toray-DuPont Co., Ltd.)
(A-13)
Aromatic ester-based polyurethane ("Miractran E574PNAT", manufactured by Nippon Polyurethane Industry Co., Ltd.)
(A-14)
Aromatic ether-based polyurethane ("Pellethane 2363-75D", manufactured by The Lubrizol Corporation)
(A-15)
Aromatic ether-based polyurethane ("Isoplast 2510", manufactured by The Lubrizol Corporation)
(A-16)
Aliphatic ether-based polyurethane ("Tecoflex EG-72D", manufactured by The Lubrizol Corporation)
(A-17)
Aliphatic carbonate-based polyurethane ("Carbothane PC-3572D", manufactured by The Lubrizol Corporation)
(A-18)
Olefin elastomer ("Santoprene 203-50", manufactured by Exxon Mobil Corporation)
(A-19)
Styrene elastomer ("Septon 2104", manufactured by Kuraray Co., Ltd.)
(A-20)
Acrylic elastomer ("Kurarity LM730H", manufactured by Kuraray Co., Ltd.) Resin Particles (P, Z)
(P-1)
Crosslinked methyl methacrylate polymer particle, particle diameter: 10 $\mu$m ("MX-1000", manufactured by Soken Chemical and Engineering Co., Ltd.)
(P-2)
Crosslinked methyl methacrylate polymer particle, particle diameter: 0.4 $\mu$m ("MX-40T", manufactured by Soken Chemical and Engineering Co., Ltd.)

(P-3)

Crosslinked methyl methacrylate polymer particle, particle diameter: 1.5 μm ("MX-150", manufactured by Soken Chemical and Engineering Co., Ltd.)

(P-4)

Crosslinked methyl methacrylate polymer particle, particle diameter: 5.0 μm ("MX-500", manufactured by Soken Chemical and Engineering Co., Ltd.)

(P-5)

Crosslinked methyl methacrylate polymer particle, particle diameter: 30 μm ("MX-3000", manufactured by Soken Chemical and Engineering Co., Ltd.)

(P-6)

Crosslinked methyl methacrylate polymer particle, particle diameter: 80 μm ("Techpolymer MBX-80", manufactured by Sekisui Plastics Co., Ltd.)

(P-7)

Crosslinked styrene polymer particle, particle diameter: 5.0 μm ("SX-500H", manufactured by Soken Chemical and Engineering Co., Ltd.)

(P-8)

Crosslinked (methyl methacrylate-styrene) polymer particle, particle diameter: 2.5 μm ("Epostar MA2003", manufactured by Nippon Shokubai Co., Ltd.)

(P-9)

Crosslinked n-butyl acrylate polymer particle, particle diameter: 8.0 μm ("Techpolymer BM30X-8", manufactured by Sekisui Plastics Co., Ltd.)

(P-10)

Thermosetting melamine particle (benzoguanamine-formaldehyde condensate), particle diameter: 9.0 μm ("Epostar L15", manufactured by Nippon Shokubai Co., Ltd.)

(Z-1)

Non-crosslinked methyl methacrylate polymer particle, particle diameter: 0.4 μm ("MP-1000", manufactured by Soken Chemical and Engineering Co., Ltd.)

(Z-2)

Crosslinked methyl methacrylate polymer particle, particle diameter: 0.07 μm ("Epostar MX050W", manufactured by Nippon Shokubai Co., Ltd.)

(Z-3)

Crosslinked methyl methacrylate polymer particle, particle diameter: 110 μm ("Art-Pearl SE-090T", manufactured by Negami Chemical Industrial Co., Ltd.)

(Z-4)

Silica particle, particle diameter: 2.5 μm ("Seahostar KE-P250", manufactured by Nippon Shokubai Co., Ltd.)

(Z-5)

Talc particle, particle diameter: 8.0 μm ("Micro Ace K-1", manufactured by Nippon Talc Co., Ltd.)

Preparation Example 1 and Comparative Preparation Example 1

Preparation of Resin Composition

The thermoplastic resin (A) and the resin particles (P, Z) were mixed in each mixing ratio presented in the Tables below and introduced into a twin-screw kneading machine (product name: "KZW15-30MG", manufactured by Technovel Corporation) heated to a temperature 20°C above the melting point of the thermoplastic resin (A). Kneading was performed at a screw rotational speed of 100 rpm to form a resin composition. The resin composition obtained through kneading was ejected from the twin-screw kneading machine and cooled in a water tank to obtain a strand. The strand was cut with a pelletizer to obtain pellets formed of the resin composition.

Preparation Example 2 and Comparative Preparation Example 2

Production of Resin Composition Sheet

The pellets obtained in Preparation Example 1 were pressure molded at a temperature 5°C above the melting point of the thermoplastic resin to obtain a resin composition sheet having a length of 100 mm, a width of 100 mm, and a thickness of 2 mm.

Test Example 1

Bending Rigidity

The bending rigidity of the resin composition sheet produced in Preparation Example 2 was measured. A No. 3 dumbbell sample was punched from the resin composition sheet and a bending test was conducted on the sample in accordance with JIS K 7171-1:2016 to determine the modulus of elasticity of the sample. The obtained modulus of elasticity was evaluated by applying the following evaluation criteria.

Bending Rigidity Evaluation Criteria

A: The modulus of elasticity is 1,000 MPa or more.
B: The modulus of elasticity is 800 MPa or more and less than 1,000 MPa.
C: The modulus of elasticity is 600 MPa or more and less than 800 MPa.
D: The modulus of elasticity is less than 600 MPa.

The results are presented in the Tables below.

Test Example 2

Hygroscopic Elongation

The strand (length: 1.0 m) obtained in Preparation Example 1 was dried at 80°C for 4 hours and then cooled to 23°C to determine the length (L0) of the strand. Subsequently, the strand was immersed in water at 23°C for 48 hours to determine the length at 23°C (L1) again. The hygroscopic elongation was determined with the following formula using L0 and L1, and the hygroscopic elongation was evaluated by applying the following evaluation criteria.

$$\text{Hygroscopic Elongation (\%)} = 100 \times (L1 - L0)/L0$$

Hygroscopic Elongation Evaluation Criteria

A: The hygroscopic elongation is less than 3%.
B: The hygroscopic elongation is 3% or more and less than 6%.
C: The hygroscopic elongation is 6% or more and less than 9%.
D: The hygroscopic elongation is 9% or more.

The results are presented in the Tables below.

Test Example 3

Ultrasonic Properties

A 5 MHz sine wave signal (wavenumber: 1) output from an ultrasonic oscillator (product name: "FG-350" function generator, manufactured by Iwatsu Measurement Co., Ltd.) was input into an ultrasound probe (manufactured by Japan Probe Co., Ltd.), and the ultrasound probe was caused to generate an ultrasonic pulse wave with a center frequency of 5 MHz in water. The amplitude of the generated ultrasonic wave before and after passing through the resin composition sheet produced in Preparation Example 2 was measured with an ultrasonic receiver ("VP-5204A" oscilloscope, manufactured by Matsushita Electric Industrial Co., Ltd.) in an environment at a water temperature of 25°C. The ultrasonic attenuation in each resin composition sheet was compared by comparing ultrasonic sensitivity. The ultrasonic sensitivity was determined to be a numerical value obtained with the following formula, where Vin represents the voltage peak value of an input wave from the ultrasonic oscillator, the input wave having a half width of 50 nsec or less; and Vs represents the voltage value obtained when the ultrasonic oscillator received the generated ultrasonic wave that had passed through the resin composition sheet and that had reflected from an opposite surface of the resin composition sheet.

$$(\text{Ultrasonic sensitivity}) = 20 \times \text{Log} \, (Vs/Vin)$$

The ultrasonic properties were evaluated by applying the following evaluation criteria to the obtained ultrasonic sensitivity.

Ultrasonic Properties Evaluation Criteria

A: The ultrasonic sensitivity is -75 dB or more.
B: The ultrasonic sensitivity is -80 dB or more and less than -75 dB.
C: The ultrasonic sensitivity is -90 dB or more and less than -80 dB.
D: The ultrasonic sensitivity is less than -90 dB.

The results are presented in the Tables below.

Test Example 4

Abrasion Resistance

The pellets of the resin composition obtained in Preparation Example 1 were dried at 80°C for a whole day and night, and the pellets were injection molded into hollow cylindrical test samples having a contact area of 2 cm$^2$ (the term "a contact area of 2 cm$^2$" refers to the area of a resin composition portion of a section of the cylinder being 2 cm$^2$). An "NS-40" injection molding machine manufactured by Nissei Plastic Industrial Co., Ltd. was used for injection molding. The cylinder temperature during injection molding was 20°C above the melting point of the thermoplastic resin, and the mold temperature was 130°C.

A test of friction and wear between the hollow cylindrical samples was conducted in accordance with the JIS K7218:1986 (A) method. The test of friction and wear was performed for 20 hours with an "EFM-III-F" wear tester manufactured by A and D Co., Ltd. in an environment at a temperature of 23°C and at a relative humidity of 50%, with a linear velocity of 100 mm/s, and with an applied load of 50 N. The specific wear amount of the test sample on a fixed side of the apparatus and the specific wear amount of the test sample on a movable side of the apparatus were respectively measured, and the total measured amount was determined to be the specific resistance of the test samples. The specific wear amount was calculated by dividing the sample volume that is reduced by wear by the total sliding distance and the applied load. The abrasion resistance was evaluated by applying the following evaluation criteria to the obtained specific wear amount of the test samples.

Abrasion Resistance Evaluation Criteria

A: The specific wear amount is less than 0.2 mm$^3$/N·km.
B: The specific wear amount is 0.2 mm$^3$/N·km or more and less than 0.6 mm$^3$/N·km.
C: The specific wear amount is 0.6 mm$^3$/N·km or more and less than 1 mm$^3$/N·km.
D: The specific wear amount is 1 mm$^3$/N·km or more.

The results are presented in the Tables below.

Table 1

| | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Thermoplastic resin (A) | Types | (A-1) | (A-2) | (A-3) | (A-4) | (A-5) | (A-6) | (A-7) | (A-8) | (A-9) | (A-10) |
| | | PA6 | PA66 | PA11 | PA12 | PA9T | PA46 | PA610 | PA1010 | Amorphous PA | PA elasto-mer |
| | Content in resin compo-sition (mass%) | 97 | 97 | 97 | 97 | 97 | 97 | 97 | 97 | 97 | 97 |
| Resin particles (P, Z) | Types | (P-1) | (P-1) | (P-1) | (P-1) | (P-1) | (P-1) | (P-1) | (P-1) | (P-1) | (P-1) |
| | | Crosslinked acrylic | Crosslinked acrylic | Crosslinked acrylic | Crosslinked acrylic | Crosslinked acrylic | Crosslinked acrylic | Crosslinked acrylic | Crosslinked acrylic | Crosslinked acrylic | Crosslinked acrylic |
| | Particle dia-meter ($\mu$m) | 10 | 10 | 10 | 10 | 10 | 10 | 10 | 10 | 10 | 10 |
| | Content in resin composition (mass%) | 3 | 3 | 3 | 3 | 3 | 3 | 3 | 3 | 3 | 3 |
| Bending rigidity | | A | A | A | A | A | A | A | A | A | A |
| Hygroscopic elongation | | B | B | A | A | B | B | B | B | A | A |
| Ultrasonic properties | | B | B | A | A | B | B | B | B | A | A |
| Abrasion resistance | | A | A | A | A | A | A | A | A | A | A |

Table 2

| | | Example 11 | Example 12 | Example 13 | Example 14 | Example 15 | Example 16 | Example 17 | Example 18 | Example 19 | Example 20 |
|---|---|---|---|---|---|---|---|---|---|---|---|
| Thermoplastic Resin (A) | Types | (A-11) | (A-12) | (A-13) | (A-14) | (A-15) | (A-16) | (A-17) | (A-18) | (A-19) | (A-20) |
| | | PA elastomer | TPEE | TPU | TPU | TPU | TPU | TPU | Olefin | Styrene | Acrylic |
| | Content in resin composition (mass%) | 97 | 97 | 97 | 97 | 97 | 97 | 97 | 97 | 97 | 97 |
| Resin particles (P, Z) | Types | (P-1) | (P-1) | (P-1) | (P-1) | (P-1) | (P-1) | (P-1) | (P-1) | (P-1) | (P-1) |
| | | Crosslinked acrylic | Crosslinked acrylic | Crosslinked acrylic | Crosslinked acrylic | Crosslinked acrylic | Crosslinked acrylic | Crosslinked acrylic | Crosslinked acrylic | Crosslinked acrylic | Crosslinked acrylic |
| | Particle diameter ($\mu$m) | 10 | 10 | 10 | 10 | 10 | 10 | 10 | 10 | 10 | 10 |
| | Content in resin composition (mass%) | 3 | 3 | 3 | 3 | 3 | 3 | 3 | 3 | 3 | 3 |
| Bending rigidity | | A | A | A | A | A | A | A | C | C | C |
| Hygroscopic elongation | | A | A | A | B | A | B | A | A | A | C |
| Ultrasonic properties | | A | C | C | C | C | C | C | A | A | A |
| Abrasion resistance | | A | B | A | A | A | A | A | A | A | B |

Table 3

| | | Example 21 | Example 22 | Example 23 | Example 24 | Example 25 | Example 26 | Example 27 | Example 28 | Example 29 | Example 30 | Example 31 |
|---|---|---|---|---|---|---|---|---|---|---|---|---|
| Thermoplastic Resin (A) | Types | (A-4) | (A-4) | (A-4) | (A-4) | (A-4) | (A-4) | (A-4) | (A-4) | (A-4) | (A-4) | (A-4) |
| | | PA12 | PA12 | PA12 | PA12 | PA12 | PA12 | PA12 | PA12 | PA12 | PA12 | PA12 |
| | Content in resin composition (mass%) | 97 | 97 | 97 | 97 | 97 | 97 | 97 | 97 | 97 | 99.5 | 98.5 |
| Resin particles (P, Z) | Types | (P-2) | (P-3) | (P-4) | (P-5) | (P-6) | (P-7) | (P-8) | (P-9) | (P-10) | (P-1) | (P-1) |
| | | Crosslinked acrylic | Crosslinked acrylic | Crosslinked acrylic | Crosslinked acrylic | Crosslinked acrylic | Crosslinked styrene | Crosslinked acrylic-styrene | Crosslinked butyl acrylate | Melamine | Crosslinked acrylic | Crosslinked acrylic |
| | Particle diameter ($\mu$m) | 0.4 | 1.5 | 5.0 | 30 | 80 | 5.0 | 2.5 | 8.0 | 9.0 | 10 | 10 |
| | Content in resin composition (mass%) | 3 | 3 | 3 | 3 | 3 | 3 | 3 | 3 | 3 | 0.5 | 1.5 |
| Bending rigidity | | A | A | A | A | A | A | A | B | A | A | A |
| Hygroscopic elongation | | A | A | A | A | A | A | A | A | A | A | A |
| Ultrasonic properties | | A | A | A | B | C | A | A | A | A | A | A |
| Abrasion resistance | | C | B | A | A | A | A | A | A | C | C | B |

Table 4

| | | Example 32 | Example 33 | Example 34 | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|---|---|---|
| Thermoplastic Resin (A) | Types | (A-4) | (A-4) | (A-4) | (A-10) | (A-11) | (A-4) | (A-4) | (A-4) | (A-4) | (A-4) | (A-4) |
| | | PA12 | PA12 | PA12 | PA elastomer | PA elastomer | PA12 | PA12 | PA12 | PA12 | PA12 | PA12 |
| | Content in resin composition (mass%) | 95 | 90 | 85 | 100 | 100 | 100 | 97 | 97 | 97 | 97 | 97 |
| Resin particles (P, Z) | Types | (P-1) | (P-1) | (P-1) | | | | (Z-1) | (Z-2) | (Z-3) | (Z-4) | (Z-5) |
| | | Crosslinked acrylic | Crosslinked acrylic | Crosslinked acrylic | | | | Non-crosslinked acrylic | Crosslinked acrylic | Crosslinked acrylic | Silica | Talc |
| | Particle diameter ($\mu$m) | 10 | 10 | 10 | None | None | None | Melted and was unable to maintain particle shape thereof | 0.07 | 110 | 2.5 | 8.0 |
| | Content in resin composition (mass%) | 5 | 10 | 15 | | | | 3 | 3 | 3 | 3 | 3 |
| Bending rigidity | | A | A | A | A | A | A | C | A | A | A | A |
| Hygroscopic elongation | | A | B | C | A | A | A | A | A | A | B | C |
| Ultrasonic properties | | A | B | C | A | A | A | D | A | D | D | D |
| Abrasion resistance | | A | A | A | D | D | D | D | D | A | D | D |

EP 3 747 368 B1

15

As presented in the Tables above, when the sheet did not include the resin fine particles, the specific wear amount increased, as indicated in the test of friction and wear, resulting in poor abrasion resistance (Comparative Examples 1 to 3). In a case where such a sheet is used as a sheath of an ultrasound probe, friction increases when the ultrasound probe is moved in an endoscope forceps opening or in body cavities.

When the resin particles were non-crosslinked acrylic resin particles, the resin particles melted and then merged with the thermoplastic resin during melt-kneading. Thus, the resin particles were unable to maintain the particle shape. In this case, the results indicated that, in the obtained sheet, the bending rigidity of polyamide 12 used as the base resin was adversely affected by the acrylic resin derived from the resin particles, and the ultrasonic properties and abrasion resistance were poor (Comparative Example 4).

Even in the case where the thermoplastic resin and the resin particles were used in combination, when the particle diameter of the resin particles was smaller than prescribed by the present invention, the results indicated poor abrasion resistance (Comparative Example 5), and, by contrast, when the particle diameter of the resin particles was larger than prescribed by the present invention, the results indicated poor ultrasonic properties (Comparative Example 6).

When inorganic particles, instead of the resin particles, were used as the particles, the results indicated poor ultrasonic properties, low compatibility with the thermoplastic resin, and poor abrasion resistance (Comparative Examples 7 and 8).

On the other hand, in all of the sheets containing a combination of the thermoplastic resin and the resin particles, the results indicated that the bending rigidity of the base resin was unaffected by the resin particles, the hygroscopic elongation was suppressed, the ultrasonic properties were excellent, and the abrasion resistance was excellent (Examples 1 to 34).

Thus, when a resin composition containing a combination of the thermoplastic resin and the resin particles is used as a sheath material for an ultrasound probe, an ultrasound probe that is capable of being moved in an endoscope forceps opening, body cavities, or the like smoothly with low friction, that is less prone to buckling while, for example, being bent sharply, that retains excellent dimensional stability after repeated washing and use, and that has excellent ultrasonic properties is obtainable.

While the present invention has been described in conjunction with embodiments thereof, we do not intend to limit our invention in any detail of the description unless otherwise specified. Rather, the invention should be broadly construed without departing from the scope of the invention as defined by the appended claims.

The present application is based on, and claims priority from JP2018-013748, filed on January 30, 2018 in Japan.

Reference Signs List

1     ultrasound probe
2     insert section
3     grip section
4     cable section
5     shaft
6     ultrasonic oscillator
7     motor
8     sheath
8a    ultrasonic transmission window
9     acoustic medium
10    acoustic lens
11    piezoelectric oscillator
12    backing layer
13    housing

## Claims

1.  An ultrasound probe (1) comprising:

    a shaft (5) having an ultrasonic oscillator (6) on a front end thereof; and
    a sheath (8) covering the shaft (5),
    wherein the sheath (8) contains a thermoplastic resin serving as a base resin including resin particles dispersed therein having a particle diameter of 0.1 to 100 $\mu$m and the material of the sheath (8) serves as a contact surface when the ultrasound probe is moved in an endoscope forceps opening or in body cavities.

**2.** The ultrasound probe (1) according to claim 1, wherein the resin particle has a particle diameter of 1 to 50 $\mu$m.

**3.** The ultrasound probe (1) according to claim 1 or 2, wherein the resin particle is formed of a resin having a crosslinked structure.

**4.** The ultrasound probe (1) according to any one of claims 1 to 3, wherein the resin particle is formed of a resin including at least one of a (meth)acrylic acid ester component or a styrene component.

**5.** The ultrasound probe (1) according to any one of claims 1 to 4, wherein the thermoplastic resin includes a polyamide resin.

**6.** The ultrasound probe (1) according to claim 5, wherein the thermoplastic resin includes at least one of polyamide 11, polyamide 12, an amorphous polyamide, or a polyamide elastomer.

**7.** The ultrasound probe (1) according to any one of claims 1 to 6, wherein a content of the resin particle in the sheath is 1% to 12% by mass.

**8.** Use of a resin composition for producing a sheath (8) covering a shaft (5), of an ultrasound probe (1), having an ultrasonic oscillator (6) on a front end thereof, wherein the resin composition comprises:
a thermoplastic resin serving as a base resin including resin particles dispersed therein having a particle diameter of 0.1 to 100 $\mu$m, and wherein the sheath material serves as a contact surface when the ultrasound probe is moved in an endoscope forceps opening or in body cavities.

**Patentansprüche**

**1.** Ultraschallsonde (1), umfassend:

einen Welle (5), die einen Ultraschalloszillator (6) an einem vorderen Ende davon aufweist; und
eine Hülle (8), die die Welle (5) abdeckt,
wobei die Hülle (8) ein thermoplastisches Harz enthält, das als ein Basisharz dient, das Harzpartikel, die darin dispergiert sind, mit einem Partikeldurchmesser von 0,1 bis 100 $\mu$m enthält,
und das Material der Hülle (8) als eine Kontaktfläche dient, wenn die Ultraschallsonde in einer Endoskop-Zangenöffnung oder in Körperhöhlen bewegt wird.

**2.** Ultraschallsonde (1) nach Anspruch 1, wobei das Harzpartikel einen Partikeldurchmesser von 1 bis 50 $\mu$m aufweist.

**3.** Ultraschallsonde (1) nach Anspruch 1 oder 2, wobei das Harzpartikel aus einem Harz mit einer vernetzten Struktur gebildet ist.

**4.** Ultraschallsonde (1) nach einem der Ansprüche 1 bis 3, wobei das Harzpartikel aus einem Harz gebildet ist, das mindestens eine von einer (Meth)acrylsäureesterkomponente und einer Styrolkomponente enthält.

**5.** Ultraschallsonde (1) nach einem der Ansprüche 1 bis 4, wobei das thermoplastische Harz ein Polyamidharz enthält.

**6.** Ultraschallsonde (1) nach Anspruch 5, wobei das thermoplastische Harz mindestens eines von Polyamid 11, Polyamid 12, einem amorphen Polyamid und einem Polyamidelastomer enthält.

**7.** Ultraschallsonde (1) nach einem der Ansprüche 1 bis 6, wobei ein Gehalt des Harzpartikels in der Hülle 1 bis 12 Massen-% beträgt.

**8.** Verwendung einer Harzzusammensetzung zum Produzieren einer Hülle (8), die eine Welle (5) einer Ultraschallsonde (1), die einen Ultraschalloszillator (6) an einem vorderen Ende davon aufweist, abdeckt, wobei die Harzzusammensetzung umfasst:

ein thermoplastisches Harz, das als ein Basisharz dient, das Harzpartikel, die darin dispergiert sind, mit einem Partikeldurchmesser von 0,1 bis 100 $\mu$m enthält,
und wobei das Hüllenmaterial als eine Kontaktfläche dient, wenn die Ultraschallsonde in einer Endoskop-Zangenöffnung oder in Körperhöhlen bewegt wird.

**Revendications**

1. Sonde ultrasonore (1) comprenant :

   un arbre (5) ayant un oscillateur ultrasonore (6) sur une extrémité avant de celui-ci ; et
   une gaine (8) recouvrant l'arbre (5),
   dans lequel la gaine (8) contient une résine thermoplastique servant de résine de base incluant des particules de résine dispersées dans celle-ci ayant un diamètre de particule de 0,1 à 100 $\mu$m
   et le matériau de la gaine (8) sert de surface de contact lorsque la sonde ultrasonore est déplacée dans une ouverture de forceps d'endoscope ou dans des cavités corporelles.

2. Sonde ultrasonore (1) selon la revendication 1, dans laquelle la particule de résine a un diamètre de particule de 1 à 50 $\mu$m.

3. Sonde ultrasonore (1) selon la revendication 1 ou la revendication 2, dans laquelle la particule de résine est formée d'une résine ayant une structure réticulée.

4. Sonde ultrasonore (1) selon l'une quelconque des revendications 1 à 3, dans laquelle la particule de résine est formée d'une résine incluant au moins l'un d'un composant d'ester d'acide (méth)acrylique ou d'un composant de styrène.

5. Sonde ultrasonore (1) selon l'une quelconque des revendications 1 à 4, dans laquelle la résine thermoplastique inclut une résine polyamide.

6. Sonde ultrasonore (1) selon la revendication 5, dans laquelle la résine thermoplastique inclut au moins l'un de polyamide 11, polyamide 12, un polyamide amorphe ou un élastomère de polyamide.

7. Sonde ultrasonore (1) selon l'une quelconque des revendications 1 à 6, dans laquelle une teneur de la particule de résine dans la gaine est de 1 % à 12 % en masse.

8. Utilisation d'une composition de résine pour produire une gaine (8) recouvrant un arbre (5) d'une sonde ultrasonore (1), ayant un oscillateur ultrasonore (6) sur une extrémité avant de celui-ci, dans laquelle la composition de résine comprend :

   une résine thermoplastique servant de résine de base incluant des particules de résine dispersées dans celle-ci ayant un diamètre de particule de 0,1 à 100 $\mu$m,
   et dans lequel le matériau de la gaine sert de surface de contact lorsque la sonde ultrasonore est déplacée dans une ouverture de forceps d'endoscope ou dans des cavités corporelles.

# FIG. 1

**EP 3 747 368 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001340342 A
- US 2014249419 A1
- US 20160338666 A1
- JP 11092636 A
- JP H1192636 A
- JP 2018013748 A